# EUROPÄISCHE PATENTANMELDUNG

(11) **EP 4 806 407 A1**
(43) Veröffentlichungstag der Anmeldung: **16.09.2026**
(21) Anmeldenummer: 25173024.8
(22) Anmeldetag: 28.04.2025
(51) Int. Cl.: A61F 13/15, A44B 18/00, A61F 13/62, B29C 65/08, B29C 65/00

(54) **VERSCHLUSSSYSTEM MIT VERSCHIEDENARTIG AUSGEBILDETEN SCHWEISSSTELLEN, VERFAHREN ZUM HERSTELLEN EINES VERSCHLUSSSYSTEMS UND HYGIENEARTIKEL MIT EINEM VERSCHLUSSSYSTEM**

(30) Priorität: 11.03.2025 DE 102025109236; 11.03.2025 DE 202025101295 U
(71) Anmelder: Gottlieb Binder GmbH & Co. KG, 71088 Holzgerlingen (DE)
(72) Erfinder: CARTER, Nick, 65191 Wiesbaden (DE); HEEPE, Lars, 72072 Tübingen (DE)
(74) Vertreter: Kohler Schmid Möbus Patentanwälte

(57) **Zusammenfassung**

Die Erfindung betrifft ein Verschlusssystem (10), ein Verfahren zum Herstellen eines Verschlusssystems (10) sowie einen Hygieneartikel (40) mit einem Verschlusssystem (10). Das Verschlusssystem (10) weist ein Trägerteil (12) mit an dessen Vorderseite (12') vorstehenden Verschlusselementen (16) und ein Festlegeteil (14), das an der Rückseite des Trägerteils (12) angeordnet ist, auf. Das Trägerteil (12) und das Festlegeteil (14) sind durch zumindest eine erste Schweißstelle (18) und zumindest eine zweite Schweißstelle (20) miteinander verbunden, wobei die Schweißstellen (18, 20) jeweils unterschiedlich ausgebildet sind. Die jeweilige unterschiedliche Ausbildung der Schweißstellen (18, 20) wird über ein Verschweißen, insbesondere mittels eines Ultraschallschweißverfahrens, mit jeweils unterschiedlichen Schweißparametern erreicht. Insbesondere können mehrere erste und/oder zweite Schweißstellen (18, 20) in Zwischenräumen (24) zwischen Reihen (17) von Verschlusselementen (16) angeordnet sein. Zweite Schweißstellen (20) können insbesondere als "Flexzonen" ausgebildet sein, wobei sie eine geringere Biegesteifigkeit als erste Schweißstellen (18) aufweisen können. Das erfindungsgemäße Verfahren eignet sich insbesondere zum Herstellen eines erfindungsgemäßen Verschlusssystems (10). Der erfindungsgemäße Hygieneartikel (40) ist insbesondere in Form einer Windel ausgestaltet und weist wenigstens ein erfindungsgemäßes Verschlusssystem (10) auf.

## Beschreibung

### Hintergrund der Erfindung

Die Erfindung betrifft ein Verschlusssystem aufweisend ein Trägerteil mit vorstehenden Verschlusselementen, die auf dessen Vorderseite angeordnet sind, und ein Festlegeteil, das an der Rückseite des Trägerteils angeordnet ist, wobei das Trägerteil und das Festlegeteil durch wenigstens eine erste Schweißstelle und wenigstens eine zweite Schweißstelle bereichsweise miteinander verbunden sind. Die Erfindung betrifft ferner ein Verfahren zum Herstellen eines solchen Verschlusssystems und einen Hygieneartikel mit einem solchen Verschlusssystem.

Zum Herstellen eines Verschlusssystems, insbesondere eines Haftverschlusssystems, wird oftmals ein Trägerteil, welches vorstehende Verschlusselemente aufweist, mit einem Festlegeteil verbunden. Das Festlegeteil kann dabei als Unterlagestruktur zur Abstützung bzw. Handhabung des Trägerteils bei der Verwendung des Verschlusssystems dienen. Darüber hinaus kann das Festlegeteil zur Anbindung des Verbundes aus Trägerteil und Festlegeteil an weitere Elemente verwendet werden. Ein Beispiel für einen derartigen Aufbau ist der Haftverschluss einer Windel, wobei ein Festlegeteil an einem "Windelohr" angebunden ist und wobei das Festlegeteil wiederum flächig mit einem Trägerteil verbunden ist, welches Verschlusselemente aufweist. Beim Anlegen der Windel können die Verschlusselemente mit einem Gegenstück verhakt werden.

Es ist bekannt ein Trägerteil und ein Festlegeteil bereichsweise miteinander zu verschweißen. EP 3 408 067 B1 und EP 2 815 733 A1 beschreiben jeweils Verfahren zum Herstellen eines Verschlusssystems, wobei ein Verschlusselemente aufweisendes Trägerteil mittels eines Ultraschallschweißverfahrens mit einem Festlegeteil verschweißt wird.

### Aufgabe der Erfindung

Es ist eine Aufgabe der Erfindung, die Handhabbarkeit von Verschlusssystemen für den Gebrauch zu verbessern, insbesondere bei zuverlässiger Fixierung eines Trägerteils an einem Festlegeteil. Es ist auch eine Aufgabe der Erfindung, ein Verfahren zum Herstellen eines Verschlusssystems bereitzustellen, insbesondere wobei ein großer Auslegungsspielraum zur Beeinflussung der Eigenschaften des hergestellten Verschlusssystems gegeben ist. Weiterhin ist es eine Aufgabe der Erfindung, einen Hygieneartikel mit einem verbesserten Verschlusssystem bereitzustellen.

### Beschreibung der Erfindung

Diese Aufgabe wird erfindungsgemäß gelöst durch ein Verschlusssystem gemäß Patentanspruch 1, ein Verfahren zum Herstellen eines Verschlusssystems gemäß Patentanspruch 14 und einen Hygieneartikel gemäß Patentanspruch 22. Die abhängigen Ansprüche geben bevorzugte Weiterbildungen wieder.

### Erfindungsgemäßes Verschlusssystem

Die Aufgabe wird mithin gelöst durch ein Verschlusssystem aufweisend ein Trägerteil mit vorstehenden Verschlusselementen, die auf dessen Vorderseite angeordnet sind, und ein Festlegeteil, das an der Rückseite des Trägerteils angeordnet ist, wobei das Trägerteil und das Festlegeteil durch wenigstens eine erste Schweißstelle und wenigstens eine zweite Schweißstelle bereichsweise miteinander verbunden sind, wobei die erste und zweite Schweißstelle durch Schweißen mit unterschiedlichen Schweißparametern erhalten sind.

Insbesondere können die unterschiedlichen Schweißparameter - in Abhängigkeit von einem jeweiligen zur Erzeugung der ersten und zweiten Schweißstelle genutzten Schweißverfahren - die zur Erzeugung der jeweiligen Schweißstelle aufgewandte Energie betreffen. Beispielsweise kann eine flächenbezogene Schweißleistung über die Variation unterschiedlicher Schweißparameter beeinflusst werden.

So können sich im Falle, dass es sich bei dem genutzten Schweißverfahren um ein Ultraschallschweißverfahren handelt, beispielsweise ein Anpressdruck und/oder eine Spaltweite zwischen einer Sonotrode und einem Amboss zur Erzeugung der ersten und zweiten Schweißstelle unterscheiden. Alternativ oder zusätzlich können beispielsweise eine Schwingungsamplitude der Sonotrode und/oder eine Ultraschallfrequenz zur Erzeugung der unterschiedlichen Schweißstellen variiert werden. Weiterhin kommt beispielsweise eine Variation einer Schweißgeschwindigkeit bzw. Schweißdauer pro Längen- oder Flächeneinheit der unterschiedlichen Schweißstellen in Frage. Alternativ oder zusätzlich kann eine unterschiedliche Nachbehandlung (z. B. Nachpressen bei noch plastischem Material der Schweißstelle) der ersten bzw. zweiten Schweißstellen erfolgen.

Bei der Anwendung eines Laserschweißverfahrens können beispielsweise die Laserleistung, die Fokuslage, ggf. die Pulslänge, das Vorgehen bei einer Schutzgaszufuhr und/oder die Schweißgeschwindigkeit variiert werden.

Die aufgeführten Schweißparameter sind nicht als abschließende Liste zu verstehen. Insbesondere können weitere Schweißparameter zur Erzeugung der unterschiedlich gearteten ersten und zweiten Schweißstelle variiert werden.

Ggf. können die erste und die zweite Schweißstelle über unterschiedliche Schweißverfahren (z. B. Laserschweißen und Ultraschallschweißen) erzeugt werden. Vorzugsweise werden sie jedoch jeweils mittels des gleichen Schweißverfahrens (z. B. beide mittels Ultraschallschweißen) erzeugt.

Insbesondere kann/können die erste und/oder die zweite Schweißstelle zumindest bis an eine Außenkante eines außerhalb einer äußersten Längsreihe von Verschlusselementen verlaufenden Randes des Trägerteils heranreichen. Derart kann erreicht werden, dass das Trägerteil am Rand nicht vom Festlegeteil absteht. Dies kann Probleme bei der Verarbeitung des Verschlusssystems vermeiden und dessen Gebrauchseigenschaften verbessern, da ein Hängenbleiben am Rand des Trägerteils vermieden wird. Zudem wird die Festigkeit, insbesondere Scherstandfestigkeit, des Verschlusssystems erhöht.

Die erste und/oder die zweite Schweißstelle kann/können insbesondere über die Außenkante des außerhalb der äußersten Längsreihe von Verschlusselementen verlaufenden Randes des Trägerelements hinausragen. Mit anderen Worten kann eine jeweilige Schweißstelle den Rand des Trägerteils derart überlappen, dass ein über die Außenkante hinausragender Bereich des Festlegeteils Teil dieser Schweißstelle ist. Dabei gibt es am über den Rand hinausreichenden Teil der entsprechenden Schweißstelle keine Verbindung zwischen Trägerteil und Festlegeteil, sondern lediglich eine Schweißung innerhalb des Festlegeteils - lediglich am im Bereich des Randes befindlichen Teil der Schweißstelle verbindet diese das Trägerteil mit dem Festlegeteil (nur dieser Bereich der tatsächlichen Verschweißung von Trägerteil und Festlegteil ist für die Schweißparameter relevant). Derart kann sichergestellt werden, dass die Außenkante des Trägerteils an der entsprechenden Schweißstelle zuverlässig mit dem Festlegeteil verbunden ist.

Wenn die erste und/oder die zweite Schweißstelle am Rand des Trägerteils angeordnet ist/sind, kann eine jeweilige Schweißstelle sich insbesondere bis über die äußerste Längsreihe von Verschlusselementen nach innen in Richtung einer Längsmittelachse des Trägerteils erstrecken. Dadurch wird die Fixierung des Randes des Trägerteils weiter verbessert.

Vorzugsweise kann ein Gegenstück Teil des Verschlusssystems sein, wobei sich das Gegenstück zum Zusammenwirken mit den Verschlusselementen eignet. Insbesondere kann das Gegenstück ein Abschnitt einer "Landing zone" zum Verschließen einer Windel sein. Das Gegenstück kann ein Vliesmaterial aufweisen. Alternativ kann das Gegenstück ebenfalls ein Verschlussteil sein, welches ein Festlegeteil und ein Trägerteil mit vorstehenden Verschlusselementen aufweist.

Die Verschlusselemente weisen bevorzugt je einen Kopf auf, der in wenigstens einer radialen Richtung über einen Stiel des Verschlusselements vorsteht. Insbesondere können die Verschlusselemente in Form von Haken, oder pilzförmig, oder palmenförmig ausgebildet sein. Die radiale Richtung bezieht sich auf eine Höhenrichtung entlang des jeweiligen Stiels.

Bevorzugt weisen das Trägerteil und/oder das Festlegeteil thermoplastisches Kunststoffmaterial, insbesondere Polyolefine, Polyester und/oder Polyamid, auf. Weiter bevorzugt weisen das Trägerteil und/oder das Festlegeteil biologisch abbaubares Kunststoffmaterial und/oder biobasiertes Kunststoffmaterial auf. Vorzugsweise unterscheidet sich wenigstens eine Materialeigenschaft der ersten und zweiten Schweißstelle voneinander. Derart kann ein lokal unterschiedliches Verhalten des Verschlusssystems erreicht werden.

Insbesondere kann sich eine Festigkeit und/oder eine Steifigkeit, insbesondere eine Biegesteifigkeit der ersten und zweiten Schweißstelle voneinander unterscheiden. Dies hat sich für die Handhabung des Verschlusssystems als besonders vorteilhaft herausgestellt.

Die Biegesteifigkeit *EI* ist grundsätzlich proportional zum Elastizitätsmodul E, zur Breite b der zu biegenden Struktur entlang der Biegeachse und zu der dritten Potenz einer Höhe *h* bzw. Dicke der zu biegenden Struktur: *EI = E(bh³*/*12),* wobei für das Flächenträgheitsmoment *I = bh³*/*12* gilt. Für mehrere lose übereinanderliegende Schichten addieren sich deren Biegesteifigkeiten zu *EIᵣₑₛ = E(b[h₁³+ h₂³]*/*12),* wenn zur Vereinfachung gleiche Elastizitätsmoduln angenommen werden. Wenn ein stoffschlüssiger Verbund übereinanderliegender Strukturen eingerichtet ist, addieren sich deren jeweilige Höhen *h₁, h₂* bzw. Dicken zu einer effektiven Höhe *h_{eff} = h₁ + h₂,* sodass eine wesentlich größere Biegesteifigkeit *EI_{eff} = E(b[h₁+h₂]³*/*12)* resultiert als bei lose übereinanderliegenden Schichten.

Weitere Beispiele für Materialeigenschaften, hinsichtlich welcher sich die Schweißstellen unterscheiden können, sind die Elastizität (maximale reversible Dehnbarkeit) bzw. Duktilität oder die Wärmeleitfähigkeit im Bereich der jeweiligen Schweißstelle. Die aufgezählten Materialeigenschaften sind beispielhaft. Sie sind nicht als abschließende Liste zu verstehen. In der Regel sind die erwähnten und weitere Materialeigenschaften zumindest teilweise miteinander gekoppelt, sodass sich die Schweißstellen insbesondere in einer Vielzahl von Materialeigenschaften unterscheiden.

Insbesondere können mithilfe der wenigstens einen sich unterscheidenden Materialeigenschaft der Schweißstellen eine oder mehrere zusätzliche vorteilhafte Eigenschaften des Verbundes aus Trägerteil und Festlegeteil hervorgerufen werden. Dies kann unter anderem eine verbesserte Biegefähigkeit bzw. Flexibilität des Verschlusssystems sein.

Beispielsweise können am Verschlusssystem mithilfe der zweiten Schweißstelle bzw. mithilfe von mehreren der zweiten Schweißstellen "Flexzonen" ausgebildet werden, welche eine geringe Biegesteifigkeit aufweisen können. Diese können somit als virtuelle Gelenke oder Scharniere bei der Handhabung des Verschlusssystems wirken. Über die "Flexzonen" kann somit festgelegt werden, wie sich das Verschlusssystem beim Angriff einer Kraft verformt. Beispielsweise kann das Verschlusssystem so ausgelegt werden, dass beim Angriff einer Kraft eine Biegung abweichend von der Kraftangriffsrichtung induziert wird. Gleichermaßen lässt sich über "Flexzonen" eine generelle (nutzersubjektive) Haptik sowie die Optik des Verschlusssystems vorteilhaft beeinflussen.

Besonders vorteilhaft ist es, wenn eine als "Flexzone" ausgebildete zweite Schweißstelle weiterhin uneingeschränkt zur Verbindung zwischen Trägerteil und Festlegeteil beiträgt und damit mehrfachen Nutzen aufweist.

Insbesondere können das Trägerteil und das Festlegeteil im Bereich der zweiten Schweißstelle ein einheitliches Verbundmaterial bilden. Die Verschweißung durchdringt dann im Bereich der zweiten Schweißstelle das Trägerteil und das Festlegteil jeweils vollständig, wobei die Materialien des Trägerteils und des Festlegteils, vorzugsweise gleichmäßig, im Verbundmaterial aufgehen.

Bei einer Betrachtung der zweiten Schweißstelle im Querschnitt bedeutet dies, dass sie größtenteils als einheitlicher "Film" mit einheitlichen Materialeigenschaften und vorzugsweise einer einheitlichen Mikrostruktur ausbildet ist. Eine Unterscheidung des Materials im Bereich der zweiten Schweißstelle in makroskopische Anteile die dem Trägerteil zugeordnet werden können und makroskopische Anteile die dem Festlegeteil zugeordnet werden können ist dabei typischerweise nicht mehr möglich. Insbesondere können sich Materialeigenschaften des einheitlichen "Films" den die zweite Schweißstelle ausbildet von Materialeigenschaften eines "Laminats" aus Festlegeteil und Trägerteil deutlich unterscheiden.

Vorzugsweise bleiben das Trägerteil und das Festlegeteil an ihren freien Oberflächen im Bereich der ersten Schweißstelle voneinander unterscheidbar.

Bei der derartigen Ausbildung der ersten Schweißstelle muss somit weniger Energie als zur Ausbildung der zweiten Schweißstelle aufgewandt werden. Vorteilhaft sind auch die resultierenden unterschiedlichen Versagensmechanismen der ersten und zweiten Schweißstelle. Beispielsweise kann das Verschlusssystem so ausgelegt werden, dass die zweite Schweißstelle bei einer Belastung die zum Versagen der ersten Schweißstelle führt unbeeinträchtigt bleibt und somit die Integrität des Verschlusssystems aufrechterhält.

Insbesondere kann sich eine Tiefe des beim Verschweißen der ersten und zweiten Schweißstelle aufgeschmolzenen bzw. plastifizierten Bereichs voneinander unterscheiden.

Vorzugsweise kann sich eine Dicke des Verbundes aus Trägerteil und Festlegeteil im Bereich der ersten und zweiten Schweißstelle voneinander unterscheiden. Derart kann das Verformungsverhalten des Verschlusssystems beeinflusst werden.

Mit der Dicke wird die maximale Ausdehnung der Schweißstellen quer zu der flächigen Ausdehnung des Trägerteils und des Festlegeteils bezeichnet (d.h. in Tiefenrichtung der jeweiligen Schweißstelle).

Die zweite Schweißstelle weist insbesondere eine Dicke auf, die mindestens 10 % geringer, bevorzugt 25 % geringer, besonders bevorzugt 50 % geringer als die Dicke der ersten Schweißstelle ist.

Vorzugsweise ist/sind die erste und/oder die zweite Schweißstelle durch Ultraschallschweißen erhalten. Wie oben bereits angedeutet, wird insbesondere bevorzugt, dass die erste und die zweite Schweißstelle durch Ultraschallschweißen erhalten sind. Ultraschallschweißen erlaubt eine rationelle Fertigung bei gleichzeitig bestehenden Anpassungsmöglichkeiten für die Schweißparameter der unterschiedlichen Schweißstellen.

Bevorzugt sind die Verschlusselemente des Trägerteils in mehreren zueinander parallel verlaufenden Reihen auf dessen Vorderseite angeordnet. Verschlusselemente unterschiedlicher Reihen können, bei einer Betrachtung quer zu den Reihen, jeweils auf gleicher Höhe angeordnet sein. Gleichermaßen können die Verschlusselemente unterschiedlicher Reihen, bei einer Betrachtung quer zu den Reihen, teilweise oder insgesamt zueinander versetzt angeordnet sein.

Die benachbarten Reihen der Verschlusselemente können insbesondere parallel zu der äußersten Längsreihe von Verschlusselementen verlaufen. Die Reihen von Verschlusselementen verlaufen dann typischerweise entlang einer Maschinenrichtung oder Vorschubrichtung im Rahmen eines Herstellverfahrens. Die äußerste Längsreihe wird, wie oben erwähnt, von den Verschlusselementen in unmittelbarer Nähe des Randes des Trägerteils gebildet. Typischerweise weist ein Trägerteil demnach zwei äußerste Längsreihen von Verschlusselementen auf - eine an jedem (Längs-)Rand.

Alternativ können die parallelen Reihen der Verschlusselemente schräg zu der äußersten Längsreihe verlaufen. Typischerweise verläuft auch in diesem Fall die äußerste Längsreihe entlang der Maschinenrichtung, während die benachbarten Reihen (zwischen zwei äußersten Längsreihen) schräg zur Maschinenrichtung verlaufen.

Insbesondere kann/können sich die erste und/oder die zweite Schweißstelle zwischen benachbarten Reihen von Verschlusselementen erstrecken, insbesondere ohne die Verschlusselemente zu beschädigen.

Vorzugsweise werden Verschlusselemente unmittelbar neben einer der Schweißstellen durch diese Schweißstelle, insbesondere bei deren Erzeugung, nicht, oder zumindest nur in vernachlässigbarem Maße, beeinträchtigt, sodass das Trägerteil des erfindungsgemäßen Verschlusssystems in diesem Bereich dieselbe Haftwirkung entfalten kann wie ein entsprechendes unverschweißt vorliegendes Trägerteil. Bei alternativen Ausführungsformen können zumindest einige Verschlusselemente beim Einbringen der ersten und/oder der zweiten Schweißstelle beschädigt (überschweißt) worden sein.

Vorzugsweise weist das Verschlusssystem eine angemessene Scherstandfestigkeit auf, wobei bei einer Verschlussfläche (entspricht der Trägerteilgrundfläche) von 625 mm² (z. B. 25 mm x 25 mm) bei einer Scherbelastung durch eine Masse von 1 kg und bei einer Umgebungstemperatur von 40° C, eine Verschlussintegrität gegenüber einem Vliesstoffgegenstück von mindestens 3 Stunden besteht.

Vorzugsweise sind mehrere voneinander beabstandete erste Schweißstellen vorhanden, und insbesondere sind mehrere voneinander beabstandete zweite Schweißstellen vorhanden.

Die jeweiligen voneinander beabstandeten Schweißstellen können insbesondere in einer Längsrichtung und/oder einer Querrichtung des Trägerteils voneinander beabstandet sein. Die Längsrichtung entspricht der Richtung des Verlaufs der äußersten Längsreihe von Verschlusselementen und damit typischerweise auch der erwähnten Maschinenrichtung.

Die mehreren ersten und/oder zweiten Schweißstellen können insbesondere in Form einer unterbrochenen Schweißlinie in einem oder mehreren Zwischenräumen zwischen unmittelbar benachbarten Reihen von Verschlusselementen angeordnet sein. Darüber hinaus können die ersten und/oder zweiten Schweißstellen, wie oben erwähnt, alternativ oder zusätzlich am Rand des Trägerteils angeordnet sein. Bei einer bevorzugten Ausführungsform sind erste und/oder zweite Schweißstellen sowohl zwischen den benachbarten Reihen von Verschlusselementen als auch am Rand des Trägerteils vorhanden.

Die ersten und/oder zweiten Schweißstellen können insbesondere eine maximale Ausdehnung (vorzugsweise in der Längsrichtung des Trägerteils) zwischen 0,1 mm und 5 mm, bevorzugt zwischen 0,2 mm und 2 mm, besonders bevorzugt zwischen 0,25 mm und 1 mm, aufweisen.

Vorzugsweise sind zumindest einige der mehreren voneinander beabstandeten ersten und/oder zweiten Schweißstellen in der Längsrichtung des Trägerteils zwischen 0,1 mm und 10 mm, bevorzugt zwischen 0,2 mm und 3 mm, besonders bevorzugt zwischen 0,3 mm und 2 mm, voneinander beabstandet.

Zusätzlich oder alternativ können in Längsrichtung unmittelbar benachbarte erste und/oder zweite Schweißstellen gegenüber einander in der Querrichtung des Trägerteils versetzt sein (einen Versatz aufweisen). Der Versatz kann insbesondere zwischen 5 % und 150 %, bevorzugt zwischen 10 % und 100 %, besonders bevorzugt zwischen 15 % und 50 %, der Ausdehnung der ersten bzw. zweiten Schweißstellen in der Querrichtung betragen.

Eine Beabstandung der zweiten Schweißstellen in Längsrichtung und ggf. ein Versatz in Querrichtung kann insbesondere vorteilhaft sein, falls die zweiten Schweißstellen als verhältnismäßig dünne "Flexzonen" ausgebildet sind. Eine Rissausbreitung durch mehrere "Flexzonen" kann damit effektiv unterbunden werden.

Bei einer Betrachtung in Querrichtung können erste und/oder zweite Schweißstellen in Längsrichtung jeweils versetzt zueinander angeordnet sein. Insbesondere liegen sie sich dabei abschnittsweise gegenüber (asynchrone Anordnung). Ggf. können die ersten und/oder zweiten Schweißstellen jeweils unterschiedliche Ausdehnungen in Längsrichtung aufweisen. Alternativ oder zusätzlich können zumindest einige erste und/oder zweite Schweißstellen bei einer Betrachtung in Querrichtung in Längsrichtung jeweils gleiche Ausdehnungen und Positionen aufweisen (synchrone Anordnung).

Auch kann eine erste und/oder zweite Schweißstelle bzw. können mehrere erste und/oder zweite Schweißstellen in der Längsrichtung am Trägerteil durchgehend ausgebildet sein.

Beispielsweise kann das Verschlusssystem zwei oder drei in Längsrichtung des Trägerteils durchgehende "Flexzonen" (zweite Schweißstellen) und mehrere in Längsrichtung und Querrichtung voneinander beabstandete erste Schweißstellen aufweisen.

Weiterhin sind Ausführungsformen denkbar wobei die ersten und zweiten Schweißstellen in der Längsrichtung des Trägerteils abwechselnd aufeinanderfolgen. Dies hat sich für den Gebrauch des Verschlusssystems als vorteilhaft erwiesen. Insbesondere können die ersten und zweiten Schweißstellen unmittelbar aneinander angrenzen. In diesem Fall berühren sich die ersten und zweiten Schweißstellen und bilden gemeinsam eine "durchgehende" Schweißlinie aus. Auch ein unmittelbares Angrenzen (Berühren) der bzw. einiger der ersten und zweiten Schweißstelle(n) in der Querrichtung des Trägerteils ist denkbar.

Bei einer bevorzugten Ausführungsvariante der Erfindung sind auf einem Abschnitt des Trägerteils, welcher entlang der äußersten Längsreihe von Verschlusselementen länger als 1 cm ist, wenigstens 0,1 %, bevorzugt wenigstens 1 %, besonders bevorzugt wenigstens 10 %, der Grundfläche des Trägerteils mit dem Festlegeteil verschweißt. Typischerweise sind auf dem Abschnitt des Trägerteils, welcher entlang der äußersten Längsreihe von Verschlusselementen länger als 1 cm ist, höchstens 40 %, bevorzugt höchstens 30 %, besonders bevorzugt höchstens 25 %, der Grundfläche des Trägerteils mit dem Festlegeteil verschweißt. Die verschweißte Fläche setzt sich aus Anteilen der ersten und zweiten Schweißstelle(n) zusammen.

### Erfindungsgemäßes Verfahren

Die Aufgabe wird auch gelöst durch ein Verfahren zum Herstellen eines Verschlusssystems, insbesondere eines Verschlusssystems in einer der oben erläuterten Ausführungen, wobei einem Trägerteil mit auf seiner Vorderseite vorstehenden Verschlusselementen auf seiner Rückseite ein Festlegteil zugeführt wird, wobei das Trägerteil und das Festlegteil durch zumindest eine erste und zumindest eine zweite Schweißstelle bereichsweise miteinander verbunden werden, und wobei die erste und zweite Schweißstelle mit unterschiedlichen Schweißparametern eingebracht werden.

Die sich unterscheidenden Schweißparameter können insbesondere aus der Gruppe der oben hinsichtlich des Verschlusssystems genannten Beispiele sein.

Vorzugsweise werden mehrere entlang der äußersten Längsreihe von Verschlusselementen beabstandete erste und/oder zweite Schweißstellen eingebracht. Die äußerste Längsreihe der Verschlusselemente verläuft, wie oben beschrieben, in einer Längsrichtung des Trägerteils. Typischerweise entspricht die Längsrichtung des Trägerteils einer Maschinenrichtung bei der Durchführung des erfindungsgemäßen Verfahrens.

Weiter bevorzugt wird die erste und/oder zweite Schweißstelle oder werden zumindest einige der ersten und/oder zweiten Schweißstellen zumindest bis an eine Außenkante des außerhalb der äußersten Längsreihe verlaufenden Randes des Trägerteils heranreichend eingebracht. Wie zuvor beschrieben, kann eine jeweilige erste oder zweite Schweißstelle insbesondere so eingebracht werden, dass sie den Rand des Trägerteils überlappt. Ein über den Rand bzw. die Außenkante des Trägerteils hinausragender Bereich des Festlegeteils kann als Teil der jeweiligen ersten oder zweiten Schweißstelle angesehen werden, wird jedoch für den Vergleich der Schweißparameter außer Betracht gelassen. Für den Vergleich der Schweißparameter wird, wie oben bereits erläutert, auf die Bereiche der tatsächlichen Verschweißung von Festlegeteil und Trägerteil abgestellt.

Eine jeweilige Schweißstelle am Rand des Trägerteils kann sich insbesondere bis über die äußerste Längsreihe von Verschlusselementen hin zu einer Längsmittelachse des Trägerteils erstrecken.

Vorzugsweise wird das erfindungsgemäße Verfahren in einem kontinuierlichen Prozess durchgeführt, wobei zumindest eine Bahn von Trägerteilmaterial mit zumindest einer Bahn von Festlegteilmaterial zusammengeführt wird und diese Bahnen miteinander verbunden werden. Ggf. kann im Anschluss das Verschlusssystem bzw. können mehrere Verschlusssysteme von der resultierenden Verbundbahn abgetrennt werden.

Während des Verbindens des Trägerteils mit dem Festlegeteil kann das Festlegeteil insbesondere gemeinsam mit dem Trägerteil relativ zu einer Schweißanlage in der Maschinenrichtung verschoben werden. Gleichermaßen kann die Schweißanlage selbst entlang der Maschinenrichtung verschoben werden. Die zu verbindenden Teile können dabei unbewegt gehalten werden oder ebenfalls verschoben werden.

Vorzugsweise beträgt eine Vorschubgeschwindigkeit entlang der äußersten Längsreihe von Verschlusselementen wenigstens 70 m/min, vorzugsweise wenigstens 100 m/min, weiter bevorzugt wenigstens 150 m/min, besonders bevorzugt wenigstens 300 m/min, ganz besonders bevorzugt wenigstens 500 m/min. Die Vorschubgeschwindigkeit bezeichnet die Geschwindigkeit mit der sich die Trägerteilbahn bzw. das Trägerteil und die Festlegeteilbahn bzw. das Festlegeteil beim Verbinden relativ zu der Schweißanlage bewegen. Falls der Vorschub nicht gleichmäßig erfolgt, bezeichnet die Vorschubgeschwindigkeit eine mittlere Vorschubgeschwindigkeit. Die Mittelung erfolgt dabei insbesondere über die zur Erzeugung eines Verschlusssystems benötigte Zeitspanne.

Insbesondere kann eine jeweilige erste und/oder zweite Schweißstelle derart eingebracht werden, dass sie sich über zumindest ein Verschlusselement hinweg erstreckt, insbesondere sodass das zumindest eine Verschlusselement beim Schweißen zerstört wird. Derart kann eine besonders feste Verbindung zwischen Festlegeteil und Trägerteil an der entsprechenden Stelle erzeugt werden.

Alternativ kann eine jeweilige erste und/oder zweite Schweißstelle derart eingebracht werden, dass sie sich zwischen benachbarten Reihen von Verschlusselementen erstreckt, insbesondere ohne die Verschlusselemente zu beschädigen. Deren Haftwirkung wird dann nicht beeinträchtigt.

Vorzugsweise werden die erste(n) und zweite(n) Schweißstelle(n) in einem Arbeitsgang eingebracht. Dies erlaubt eine besonders rationelle Fertigung.

Besonders bevorzugt können die erste(n) und die zweite(n) Schweißstelle(n) jeweils mithilfe desselben Werkzeugs der Schweißanlage eingebracht werden. Ggf. kann die Erzeugung einer ersten und einer zweiten Schweißstelle zeitgleich erfolgen.

Alternativ kann vorgesehen sein, dass die erste(n) und zweite(n) Schweißstelle(n) nacheinander erzeugt werden.

Beispielsweise können zunächst mehrere erste Schweißstellen erzeugt werden und anschließend mehrere zweite Schweißstellen eingebracht werden. Dabei können ggf. zweite Schweißstellen über zuvor eingebrachte erste Schweißstellen hinweg erzeugt werden bzw. ein Teil der zuvor einbrachten ersten Schweißstellen zu zweiten Schweißstellen (durch erneutes Bearbeiten) modifiziert werden. Umgekehrt ist auch ein Einbringen von ersten Schweißstellen nach dem Erzeugen der zweiten Schweißstellen denkbar.

Vorzugsweise werden die erste(n) und zweite(n) Schweißstelle(n) durch Ultraschallschweißen eingebracht, insbesondere wobei eine Sonotrode an dem Festlegeteil anliegt und ein Amboss, insbesondere ein rotierender, walzenförmiger Amboss, an dem Trägerteil anliegt.

Der Amboss kann damit insbesondere an der Verschlusselemente tragenden Vorderseite des Trägerteils anliegen. Ein rotierender, walzenförmiger Amboss ermöglicht besonders große Vorschubgeschwindigkeiten. Die Sonotrode kann vorzugsweise plattenförmig ausgebildet sein. Bei entsprechender Dimensionierung kann die Sonotrode ggf. ebenfalls walzenförmig ausgebildet sein.

Insbesondere kann der Amboss wenigstens einen ersten und wenigstens einen zweiten Schweißvorsprung zum Erzeugen der ersten und zweiten Schweißstelle aufweisen.

Vorzugsweise kann wenigstens einer der Schweißvorsprünge in einen Zwischenraum zwischen unmittelbar benachbarten Reihen von Verschlusselementen eingreifen. Insbesondere kann der Schweißvorsprung deren radial vorstehende Köpfe zumindest teilweise untergreifen. Beim Einführen des betreffenden Schweißvorsprungs in den Zwischenraum kann ggf. ein geringfügiges Verdrängen der Verschlusselemente erfolgen, um das Untergreifen zu ermöglichen.

Zur Erzeugung der unterschiedlichen Schweißparameter für die erste und zweite Schweißstelle kann sich insbesondere eine Höhe des ersten und zweiten Schweißvorsprungs voneinander unterscheiden bzw. ein Radius des ggf. walzenförmigen Ambosses kann sich im Bereich des ersten und zweiten Schweißvorsprungs unterscheiden.

Insbesondere kann dadurch bedingt ein Schweißspalt zwischen dem Amboss und der Sonotrode im Bereich der ersten und zweiten Schweißstelle unterschiedlich weit sein.

Alternativ kann eine Variation des Schweißspaltes mittels anderer Maßnahmen hervorgerufen werden. Beispielsweise könnten sich zusätzliche Vorsprünge und/oder Vertiefungen an der Sonotrode befinden.

Es ist denkbar, dass ein Schweißvorsprung eine sich ändernde radiale Höhe aufweist. Insbesondere kann ein fließender Übergang von der ersten zu der zweiten Schweißstelle realisiert werden. Bei einem gestuften Schweißvorsprung können die unterschiedlichen Schweißstellen unmittelbar aneinander angrenzen.

Unabhängig von der Ausgestaltung des Ambosses/der Sonotrode bzw. des Schweißspalts können, ggf. zusätzlich, andere Schweißparameter variiert werden, um die erste und die zweite Schweißstelle zu erzeugen. Beispielsweise kann die Amplitude und/oder Frequenz der Sonotrode intermittierend geändert werden.

Bei einer besonders bevorzugten Verfahrensvariante weist der walzenförmige, rotierende Amboss an seinem Umfang jeweils mehrere erste und/oder zweite Schweißvorsprünge auf. Die mehreren ersten Schweißvorsprünge bzw. die mehreren zweiten Schweißvorsprünge können insbesondere in Umfangsrichtung voneinander beabstandet sein. Derart ergibt sich beim Abrollen des Ambosses auf dem Trägerteil eine definierte Abfolge von Kontakten der jeweiligen Schweißvorsprünge mit dem Trägerteil und ein daraus folgendes Verschweißen.

Bei einer weiter bevorzugten Variante des Verfahrens wird zugleich eine Mehrzahl von Trägerteilen bzw. Trägerteilbahnen mit wenigstens einem Festlegeteil bzw. wenigstens einer Festlegeteilbahn im Rahmen einer Parallelbearbeitung verschweißt. Gleichermaßen können insbesondere jeweils ein (einziges) Trägerteil oder mehrere Trägerteile mit mehreren Festlegeteilen im Rahmen einer Parallelbearbeitung an einer Schweißanlage bearbeitet werden.

### Erfindungsgemäßer Hygieneartikel

Die Aufgabe wird weiterhin gelöst durch einen Hygieneartikel, insbesondere in Form einer Windel, mit einem Verschlusssystem in einer der oben beschriebenen Ausführungsvarianten. Insbesondere kann das Verschlusssystem mittels des zuvor beschriebenen Verfahrens hergestellt worden sein.

Besonders vorteilhaft an einem derartigen Hygieneartikel ist, dass ein Trägerteil des Verschlusssystems zuverlässig an einem Festlegeteil angebracht ist, wobei Handhabung des Verschlusssystems und Tragegefühl des Hygieneartikels insbesondere durch die unterschiedlichen Ausgestaltungen der zumindest einen ersten und der zumindest einen zweiten Schweißstelle in vorteilhafter Weise beeinflusst werden.

Weitere Merkmale und Vorteile der Erfindung ergeben sich aus der Beschreibung, den Ansprüchen und der Zeichnung. Erfindungsgemäß können die vorstehend genannten und die noch weiter ausgeführten Merkmale jeweils einzeln für sich oder zu mehreren in beliebigen, zweckmäßigen Kombinationen Verwendung finden. Die gezeigten und beschriebenen Ausführungsformen sind nicht als abschließende Aufzählung zu verstehen, sondern haben vielmehr beispielhaften Charakter für die Schilderung der Erfindung.

### Kurzbeschreibung der Zeichnung

Die Erfindung ist in der Zeichnung dargestellt und wird anhand von Ausführungsbeispielen beschrieben. Es zeigen:
- Fig. 1a: eine perspektivische Ansicht einer ersten Ausführungsform eines erfindungsgemäßen Verschlusssystems;
- Fig. 1b: eine Draufsicht auf das Verschlusssystem aus Fig. 1a;
- Fig. 1c: eine Draufsicht auf eine Bahn mit mehreren erfindungsgemäßen Verschlusssystemen, wie sie in den Fign. 1a und 1b einzeln dargestellt sind;
- Fig. 1d: eine ausschnittsweise Seitenansicht des Verschlusssystems aus den Fign. 1a - 1c;
- Fig. 2: eine zweite Ausführungsform eines erfindungsgemäßen Verschlusssystems in einer Draufsicht;
- Fig. 3: eine dritte Ausführungsform eines erfindungsgemäßen Verschlusssystems in einer Draufsicht;
- Fig. 4a: eine vierte Ausführungsform eines erfindungsgemäßen Verschlusssystems in einer Draufsicht;
- Fig. 4b: eine fünfte Ausführungsform eines erfindungsgemäßen Verschlusssystems in einer Draufsicht;
- Fig. 4c: eine sechste Ausführungsform eines erfindungsgemäßen Verschlusssystems in einer Draufsicht;
- Fig. 4d: eine siebte Ausführungsform eines erfindungsgemäßen Verschlusssystems in einer Draufsicht;
- Fig. 4e: eine achte Ausführungsform eines erfindungsgemäßen Verschlusssystems in einer Draufsicht;
- Fig. 4f: eine neunte Ausführungsform eines erfindungsgemäßen Verschlusssystems in einer Draufsicht;
- Fig. 5a: eine perspektivische Ansicht einer Ultraschallschweißanlage zur Herstellung eines erfindungsgemäßen Verschlusssystems bzw. zur Durchführung eines erfindungsgemäßen Verfahrens;
- Fig. 5b: eine ausschnittweise, teilweise geschnittene Detailansicht der Ultraschallschweißanlage aus Fig. 5a;
- Fig. 6: einen erfindungsgemäßen Hygieneartikel in Form einer Windel mit zwei erfindungsgemäßen Verschlusssystemen.

### Detaillierte Beschreibung der Erfindung und Zeichnung

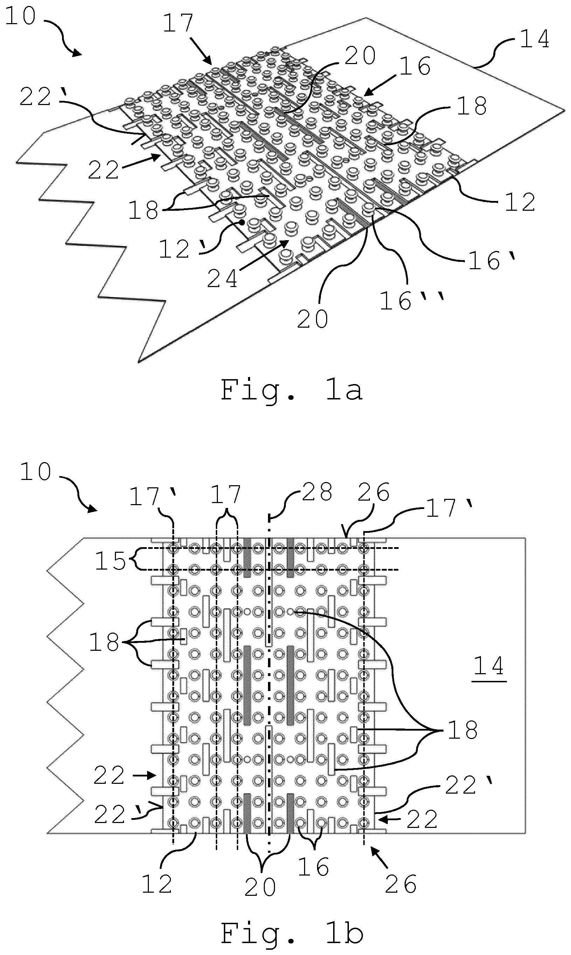

**Fig. 1a** zeigt eine perspektivische Ansicht eines Verschlusssystems **10,** welches ein Trägerteil **12** und ein Festlegeteil **14** aufweist. Das Trägerteil 12 weist an seiner Vorderseite **12'** vorstehende Verschlusselemente **16** auf. (In den Figuren der Zeichnung sind mehrfach auftretende Elemente zur besseren Übersichtlichkeit i. d. R. nur zum Teil mit Bezugszeichen versehen.) Die Verschlusselemente 16 sind im dargestellten Ausführungsbeispiel in Form von Pilzen ausgebildet, wobei ein jeweiliger Pilz in einen Kopf **16'** und einen Stiel **16"** unterteilt werden kann, wobei der Kopf 16' in radialer Richtung über den Stiel 16" auskragt.

Das Trägerteil 12 ist an ersten Schweißstellen **18** sowie an zweiten Schweißstellen **20** mit dem Festlegeteil 14 verbunden. Einige der ersten Schweißstellen 18 befinden sich hier jeweils zwischen zwei unmittelbar benachbarten Verschlusselementen 16 an einem Rand **22** des Trägerteils 12, wobei sie sich insbesondere bis über eine Außenkante **22'** des Randes 22 auf das Festlegeteil 14 erstrecken. Weitere erste Schweißstellen 18 erstrecken sich in Zwischenräumen **24** zwischen Reihen **17** (siehe Fig. 1b) von unmittelbar benachbarten Verschlusselementen 16. Die zweiten Schweißstellen 20 erstrecken sich bei diesem Ausführungsbeispiel ebenfalls in den Zwischenräumen 24 zwischen den Reihen 17.

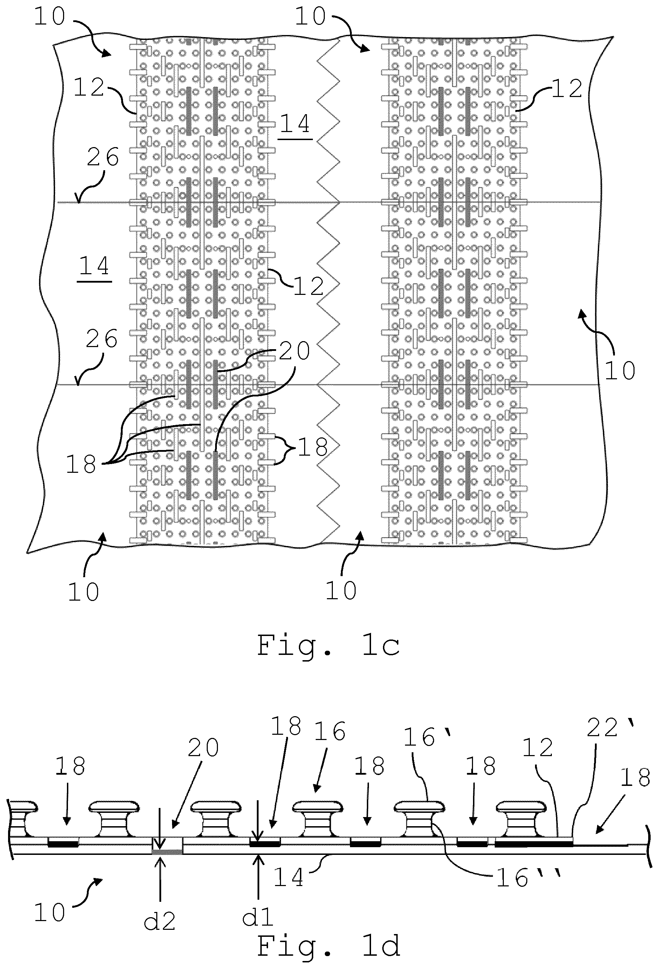

Die ersten Schweißstellen 18 und die zweiten Schweißstellen 20 sind durch Schweißen mit unterschiedlichen Schweißparametern erhalten. Die zweiten Schweißstellen 20 weisen insbesondere eine geringere Dicke (vgl. Fig. 1d) und eine geringere Biegesteifigkeit als die ersten Schweißstellen 18 auf.

Die Anzahl sowie Größe der Verschlusselemente 16 des Verschlusssystems 10 kann grundsätzlich frei gewählt werden. Bei einer Ausbildung der Verschlusselemente 16 in Form von Pilzen wird vorzugsweise eine größere Anzahl und geringere Größe der Verschlusselemente 16 gewählt als in Fig. 1a dargestellt ist. Die Darstellung der Verschlusselemente 16 und der Schweißstellen 18 sowie der zusätzlichen Schweißstellen 20 ist zur besseren Übersichtlichkeit in den Figuren der Zeichnung im Vergleich zur Darstellung des Festlegeteils 14 insbesondere überdimensioniert und rein schematisch zu verstehen.

**Fig. 1b** zeigt das Verschlusssystem 10 aus Fig. 1a in einer Draufsicht. Dabei wird die Anordnung der ersten Schweißstellen 18 und der zweiten Schweißstellen 20 deutlich. Die zweiten Schweißstellen 20 sind dunkler dargestellt. Sie erstrecken sich in zweien der Zwischenräume 24 (siehe Fig. 1a) zwischen Paaren von unmittelbar benachbarten Reihen 17 zu beiden Seiten einer Längsmittelachse **28** des Trägerteils 12. Zwischen zwei der zweiten Schweißstellen 20 ist bei einer Betrachtung in Längsrichtung des Trägerteils 12 jeweils eine erste Schweißstelle 18 (hier als Schweißpunkt ausgebildet) angeordnet.

Die Verschlusselemente 16 sind hier beispielhaft vollständig regelmäßig angeordnet, sodass neben den Reihen 17 auch Querreihen **15** ausgebildet sind. Darüber hinaus verlaufen die Reihen 17 parallel zu einer jeweiligen äußersten Längsreihe **17',** sodass die jeweilige äußerste Längsreihe 17' gleichermaßen eine Reihe 17 darstellt. Die Erstreckung der äußersten Längsreihe 17' entspricht der Längsrichtung des Trägerteils 12.

Einige der ersten Schweißstellen 18 sind entlang der Ränder 22 des Trägerteils 12 zueinander beabstandet angeordnet und überlappen die jeweilige Außenkante 22' zum Festlegeteil 14 hin. An Querrändern **26** des Verschlusssystems 10 können die dort vorhandenen ersten Schweißstellen 18 und die dort vorhandenen zweiten Schweißstellen 20 bündig mit dem Trägerteil 12 abschließen. Bei dem hier dargestellten Ausführungsbeispiel sind Verschlusselemente 16 weder durch die ersten Schweißstellen 18 noch durch die zweiten Schweißstellen 20 beeinträchtigt.

Die zweiten Schweißstellen 20 verlaufen im dargestellten Ausführungsbeispiel bei einer Betrachtung entlang der Querreihen 15 (d. h. in Querrichtung des Trägerteils 12) vollständig auf gleicher Höhe (synchrone Anordnung).

**Fig. 1c** zeigt eine Mehrzahl gleichartiger Verschlusssysteme 10 in einer Draufsicht. Aus Fig. 1c ist ersichtlich, dass die Verschlusssysteme 10 in einem kontinuierlichen Prozess aus zumindest einem Band, hier mehreren Bändern, des Trägerteils 12 und hier einem Band, ggf. mehreren Bändern, des Festlegeteils 14 hergestellt werden. Das erhaltene Verbundband kann anschließend durchtrennt werden, um die mehreren Verschlusssysteme 10 zu erhalten. Bevorzugt erfolgt das Durchtrennen an den Querrändern 26 der Verschlusssysteme 10, wobei dort vorhandene erste Schweißstellen 18 und zweite Schweißstellen 20 insbesondere etwa halbiert werden können. Dadurch kann ein sicherer Halt des jeweiligen Trägerteils 12 auf dem jeweiligen Festlegeteil 14 auch an den Querrändern 26 erreicht werden.

**Fig. 1d** zeigt ausschnittweise eine Seitenansicht des Verschlusssystems 10 aus den Fign. 1a und 1b. Die Längsrichtung des Trägerteils 12 erstreckt sich in die Darstellungsebene hinein. Das Trägerteil 12 trägt die Verschlusselemente 16, welche pilzförmig ausgebildet sind und je einen Kopf 16' und einen Stiel 16" aufweisen.

Das Trägerteil 12 ist mittels der ersten Schweißstellen 18 und der zweiten Schweißstellen 20 mit dem Festlegeteil 14 verbunden. Eine der dargestellten ersten Schweißstellen 18 überlappt die Außenkante 22' des Trägerteils 12 zum Festlegeteil 14 hin. In Fig. 1d ist lediglich eine der zweiten Schweißstellen 20 sichtbar.

Im Bereich der ersten Schweißstellen 18 weist das Verschlusssystem 10 senkrecht zu den flächigen Ausdehnungen von Trägerteil 12 und Festlegeteil 14 eine erste Dicke **d1** auf. Im Bereich der zweiten Schweißstelle 20 weist das Verschlusssystem 10 demgegenüber eine geringere zweite Dicke **d2** auf.

Im dargestellten Querschnitt der zweiten Schweißstelle 20 bilden das Trägerteil 12 und das Festlegeteil 14 ein einheitliches Verbundmaterial (einen einheitlichen "Film") aus. Die zweite Dicke d2 des Verbunds aus Trägerteil 12 und Festlegeteil 14 entspricht der Dicke der zweiten Schweißstelle 20. Der beim Verschweißen aufgeschmolzene Bereich erstreckt sich über die gesamte zweite Dicke d2.

Das Flächenträgheitsmoment *I₂₀* im Bereich einer zweiten Schweißstelle 20 ist insofern proportional zur dritten Potenz der zweiten Dicke d2. Zudem ist die Biegesteifigkeit proportional zum resultierenden Elastizitätsmodul E_{eff} des Verbundmaterials. Insgesamt ergibt sich für die Biegesteifigkeit folgende Proportionalität: *E_{eff}I₂₀ ~ E_{eff} * [d2]³.*

Im Gegensatz dazu sind die ersten Schweißstellen 18 in der Art eines "Laminats" ausgebildet, wobei im Bereich der ersten Schweißstellen 18 das Trägerteil 12 und das Festlegeteil 14 an ihren freien Oberflächen voneinander unterscheidbar bleiben. Die Dicke d1 des Verbunds ist größer als die Ausdehnung der materialschlüssigen Verbindung im Bereich der ersten Schweißstellen 18. Die Tiefe des beim Verschweißen aufgeschmolzenen Bereichs ist kleiner als die erste Dicke d1. Die Tiefe des beim Verschweißen aufgeschmolzenen Bereichs der ersten Schweißstellen 18 kann kleiner oder größer sein als die Dicke d2 im Bereich der zweiten Schweißstellen 20. Insbesondere wird im Bereich der ersten Schweißstellen 18 das Festlegteil 14 an seiner vom Trägerteil 12 abgewandten Oberfläche nicht aufgeschmolzen. Das Trägerteil 12 kann im Bereich der ersten Schweißstellen 18 durchgängig aufgeschmolzen werden.

Das Flächenträgheitsmoment *I₁₈* im Bereich einer ersten Schweißstelle 18 ist dabei proportional zur dritten Potenz der Dicke d1 des Laminats (umfassend die Dicke des verschweißten Bereichs im engeren Sinne und ggf. die darüber liegenden, beim Verschweißen nicht aufgeschmolzenen Bereiche des Trägerteils 12 und/oder des Festlegeteils 14). Somit gilt *I₁₈ ~ [d1]³ >> I₂₀ ~ [d2]³.* Ein entsprechendes Verhältnis gilt für die Biegesteifigkeit, wenn die Elastizitätsmoduln des Schweißverbundes und ggf. nicht aufgeschmolzener Bereiche des Festlegteils 14 bzw. Trägerteils 12 in derselben Größenordnung liegen. Sofern sich die Elastizitätsmoduln der beim Verschweißen der ersten Schweißstellen 18 nicht aufgeschmolzenen Bereiche des Festlegteils 14 bzw. Trägerteils 12 signifikant vom Elastizitätsmodul des Schweißverbunds unterscheiden sollten, beeinflusst dies die resultierende Biegesteifigkeit entsprechend, wobei typischerweise die Dickenabhängigkeit aufgrund der dritten Potenz deutlich überwiegt.

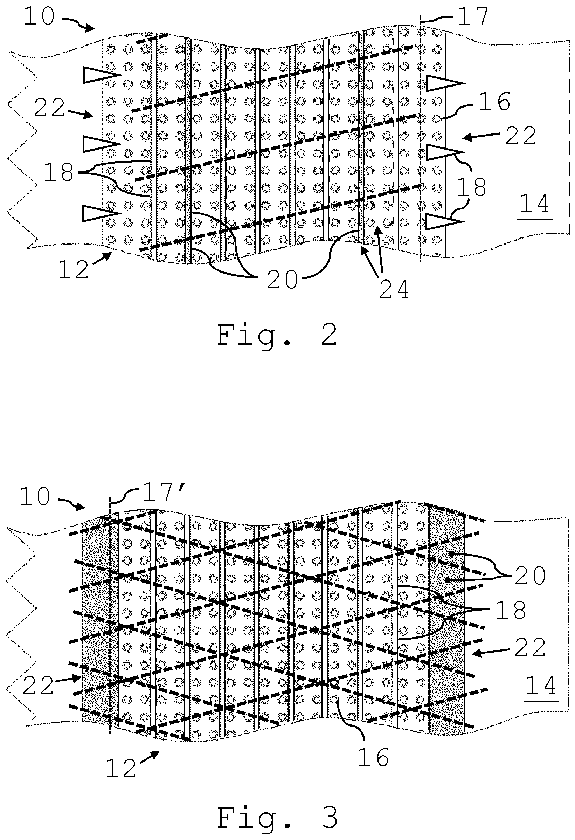

**Fig. 2** zeigt ausschnittsweise eine Ausführungsform eines Verschlusssystems 10 bei dem erste Schweißstellen 18 und zweite Schweißstellen 20 in je einem Zwischenraum 24 zwischen zwei jeweiligen unmittelbar benachbarten Reihen 17 von Verschlusselementen 16 nur einen geringen Abstand zueinander aufweisen. "Schweißlinien" sind dabei zur Ausbildung von diskreten ersten und zweiten Schweißstellen 18, 20 eines Zwischenraumes 24 entlang der in Fig. 2 dargestellten diagonal verlaufenden gestrichelten Linien unterbrochen. Die jeweiligen Unterbrechungen weisen nur geringe Ausdehnungen auf und sind nicht im Detail dargestellt.

Erste Schweißstellen 18 befinden sich zudem an Rändern 22 eines Trägerteils 12. Die ersten Schweißstellen 18 an den Rändern 22 sind hier als Dreiecke ausgebildet. Die Orientierung verschiedener Dreiecke ist im dargestellten Ausführungsbeispiel beidseits gleich, wodurch sich aus Sicht des Trägerteils 12 eine unterschiedliche Schweißgeometrie der ersten Schweißstellen 18 an den beiden Rändern 22 ergibt. Am rechten Rand 22 erstrecken sich erste Schweißstellen 18 insbesondere über Verschlusselemente 16 hinweg (die Verschlusselemente 16 wurden überschweißt).

**Fig. 3** zeigt ausschnittsweise eine Ausführungsform eines Verschlusssystems 10 bei dem jeweils mehrere erste und zweite Schweißstellen 18, 20 ähnlich zu der Darstellung in Fig. 2 durch kurze Unterbrechungen von "Schweißlinien" an den diagonal verlaufenden gestrichelten Linien ausgebildet werden. Die zweiten Schweißstellen 20 befinden sich im dargestellten Ausführungsbeispiel jeweils an Rändern 22 eines Trägerteils 12, wobei eine jeweilige äußerste Längsreihe 17' von (ehemaligen) Verschlusselementen 16 komplett überschweißt ist.

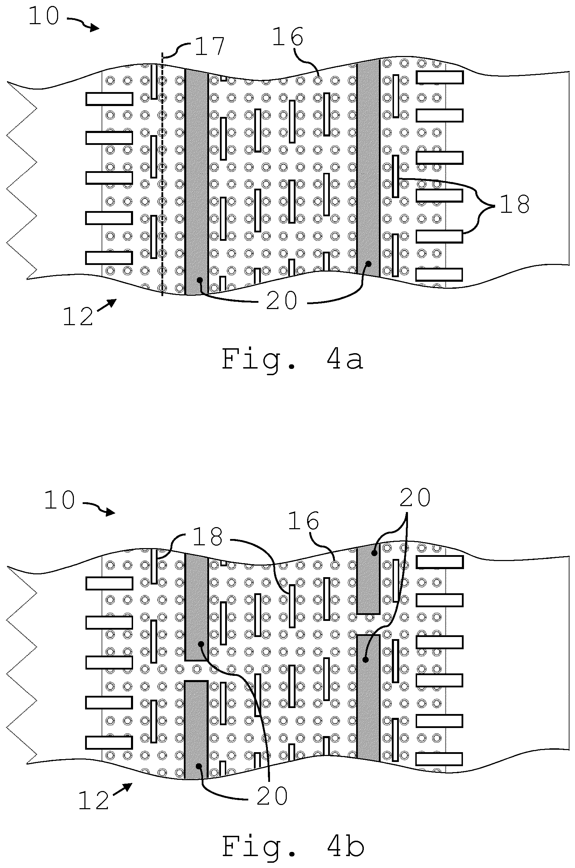

Fign. 4a - 4f zeigen ausschnittsweise jeweils weitere Ausführungsformen eines Verschlusssystems 10. Das Verschlusssystem 10 weist in den Fign. 4a - 4f jeweils die gleiche Konfiguration von ersten Schweißstellen 18 auf. Die Anordnung von zweiten Schweißstellen 20 unterscheidet sich jeweils.

Insbesondere sind zweite Schweißstellen 20 in **Fig. 4a** als in Längsrichtung eines Trägerteils 12 durchgehende Schweißlinien ausgebildet. Die zweiten Schweißstellen 20 überdecken dabei jeweils eine Reihe 17 von ursprünglich vorhandenen Verschlusselementen 16 des Trägerteils 12. Die überdeckten Verschlusselemente 16 sind praktisch nicht mehr vorhanden, da überschweißt. Die zweiten Schweißstellen 20 können insbesondere als zuvor beschriebene "Flexzonen" geringer Dicke ausgebildet sein.

In **Fig. 4b** sind mehrere zweite Schweißstellen 20 insbesondere in Längsrichtung eines Trägerteils 12 voneinander beabstandet. Bei einer Betrachtung in Querrichtung verlaufen die jeweiligen Unterbrechungen zwischen den in Längsrichtung beabstandeten zweiten Schweißstellen 20 nicht auf derselben Höhe. Es handelt sich um eine asynchrone Anordnung der zweiten Schweißstellen 20. Die in Fig. 4b dargestellten zweiten Schweißstellen 20 erstrecken sich analog zur Darstellung in Fig. 4a über einige der ursprünglich am Trägerteil 12 vorhandenen Verschlusselemente 16 hinweg.

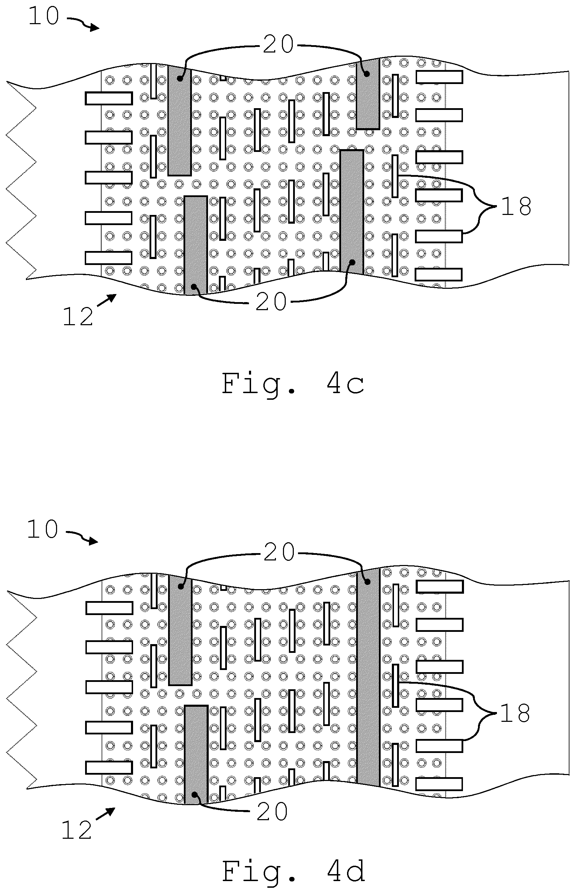

In **Fig. 4c** weisen zweite Schweißstellen 20 wiederum eine Beabstandung in Längsrichtung eines Trägerteils 12 sowie eine asynchrone Anordnung auf. Zusätzlich weisen die in Längsrichtung benachbarten zweiten Schweißstellen 20 einen Versatz in Querrichtung zueinander auf.

**Fig. 4d** zeigt ein weiteres Ausführungsbeispiel eines Verschlusssystems 10, wobei unterschiedliche Ausbildungen von zuvor beschriebenen zweiten Schweißstellen 20 miteinander kombiniert sind. Eine zweite Schweißstelle 20 ist in Längsrichtung durchgehend ausgebildet; andere zweite Schweißstellen sind in Längsrichtung voneinander beabstandet und in Querrichtung gegeneinander versetzt.

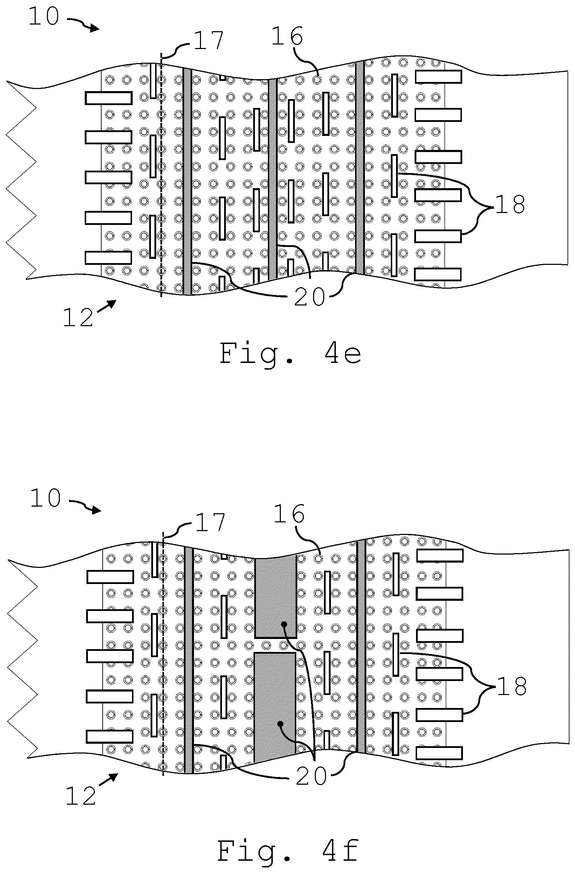

In **Fig. 4e** ist eine Verschlusssystem 10 mit drei zweiten Schweißstellen 20 abgebildet, welche jeweils in Längsrichtung eines Trägerteils 12 als durchgehende Schweißlinien ausgebildet sind. Die zweiten Schweißstellen 20 sind in Fig. 4e zwischen unmittelbar benachbarten Reihen 17 von Verschlusselementen 16 angeordnet.

**Fig. 4f** zeigt eine weitere Ausführungsform eines Verschlusssystems 10, wobei zwei zweite Schweißstellen 20 jeweils in Längsrichtung eines Trägerteils 12 als durchgehende Schweißlinien zwischen unmittelbar benachbarten Reihen 17 von Verschlusselementen 16 ausgebildet sind. Weitere zweite Schweißstellen 20 sind in Längsrichtung des Trägerteils 12 voneinander beabstandet und überdecken jeweils mehrere der ursprünglich vorhandenen Verschlusselemente 16 des Trägerteils 12.

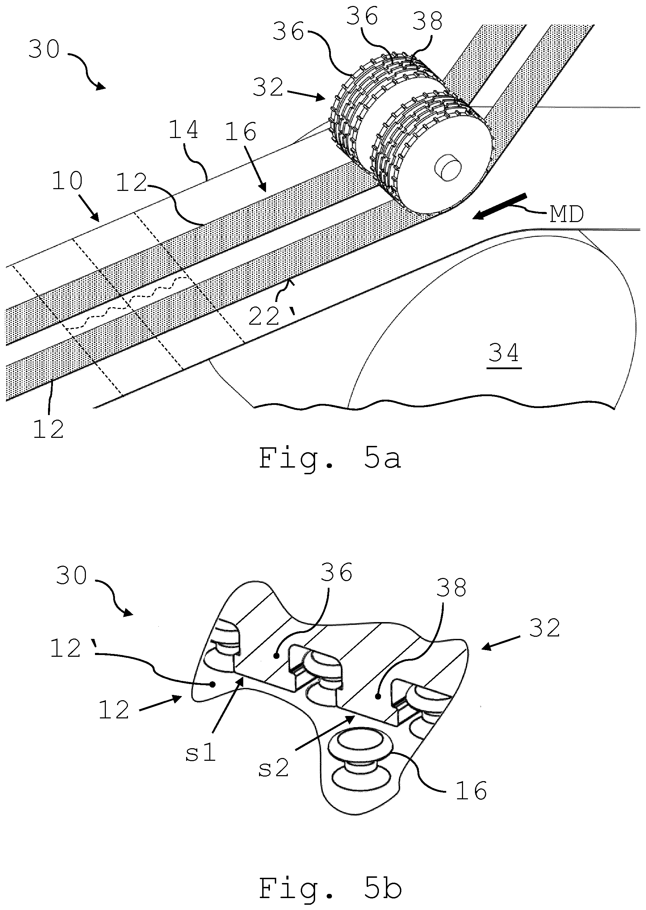

**Fig. 5a** zeigt schematisch eine perspektivische, ausschnittsweise Ansicht einer Ultraschallschweißanlage **30,** welche einen rotierenden, walzenförmigen Amboss **32** und eine Sonotrode **34** aufweist. Sonotrode 34 und Amboss 32 wirken zur Herstellung einer jeweiligen ersten und zweiten Schweißstelle 18, 20 (siehe Fign. 1a - 4f) zusammen. Fig. 5 zeigt ein bandförmiges Festlegeteil 14 sowie zwei bandförmige Trägerteile 12, welche in einer Maschinenrichtung MD zusammengeführt und miteinander verschweißt werden. Über gestrichelte Linien ist angedeutet, dass durch Zuschneiden einzelne Verschlusssysteme 10 erhalten werden können. Ein Zuschneiden muss nicht notwendigerweise wie angedeutet erfolgen. Insbesondere kann ein Verschlusssystem 10 beispielsweise mehrere Trägerteile 12 auf einem Festlegeteil 14 aufweisen.

Der Amboss 32 weist diskrete erste Schweißvorsprünge **36** zur Erzeugung der ersten Schweißstellen 18 auf. Die ersten Schweißvorsprünge 36 greifen beim Verschweißen eines Trägerteils 12 mit einem Festlegeteil 14 insbesondere zumindest teilweise in Zwischenräume 24 zwischen unmittelbar benachbarten Reihen 17 von Verschlusselementen 16 ein (vergleiche Fign. 1a - 4f).

Der Amboss 32 weist weiterhin zweite Schweißvorsprünge **38** zur Erzeugung von zweiten Schweißstellen 20 auf.

Der lokale Radius des Ambosses 32 ist an den zweiten Schweißvorsprüngen 38 insbesondere größer als an den ersten Schweißvorsprüngen 36. Dadurch wird ein jeweiliger Schweißspalt bei der jeweiligen Erzeugung der ersten Schweißstellen 18 und der zweiten Schweißstellen 20 verändert, wobei in der Folge erste und zweite Schweißstellen 18, 20 mit jeweils unterschiedlichen Schweißparametern erzeugt werden.

Der Amboss 32 kann als einstückiges Bauteil vorliegen oder mehrere Komponenten aufweisen. Insbesondere kann der Amboss 32 modular erweitert oder abgeändert werden, beispielsweise um unterschiedlich viele Bahnen von Trägerteilen 12 gleichzeitig zu bearbeiten. Bevorzugt lässt sich der Amboss 32 einfach durch einen anders gestalteten Amboss 32 austauschen, beispielsweise um das Muster der jeweiligen ersten und zweiten Schweißstellen 18, 20 zu verändern. Mittels des in Fig. 5a dargestellten Prinzips lassen sich insbesondere hohe Prozessgeschwindigkeiten bei der Durchführung eines erfindungsgemäßen Verfahrens zum Herstellen eines Verschlusssystems 10 erreichen.

**Fig. 5b** zeigt ausschnittsweise eine geschnittene Ansicht des Ambosses 32 der Ultraschallschweißanlage 30 aus Fig. 5a mit einem der ersten Schweißvorsprünge 36 und einem der zweiten Schweißvorsprünge 38. Die Schweißvorsprünge 36, 38 wirken an der Vorderseite 12' des Trägerteils 12 mit der unter dem Trägerteil 12 und dem Festlegeteil 14 (siehe Fig. 5a) angeordneten Sonotrode 34 (siehe Fig. 5a) zur Erzeugung der jeweiligen ersten und zweiten Schweißstellen 18, 20 (siehe Fign. 1a - 4f) zusammen.

Insbesondere dringt der zweite Schweißvorsprung 38 zur Erzeugung der zweiten Schweißstelle 20 weiter in Richtung der Vorderseite 12' des Trägerteils 12 vor als der erste Schweißvorsprung 36 zur Erzeugung der ersten Schweißstelle 18. Die walzenförmige Sonotrode 34 weist hier einen einheitlichen Radius auf; ihre Mantelfläche ist in diesem Ausführungsbeispiel im für die Verschweißung wirksamen Bereich glatt, d. h. ohne Erhöhungen oder Vertiefungen ausgebildet. Dadurch ist ein erster Schweißspalt **s1** im dargestellten Ausführungsbeispiel weiter als ein zweiter Schweißspalt **s2.** Entsprechend ergibt sich im Bereich des ersten Schweißspalts s1 eine größere Biegesteifigkeit nach dem Verschweißen als im Bereich des zweiten Schweißspalts s2.

Die Schweißvorsprünge 36, 38 greifen im dargestellten Ausführungsbeispiel zwischen benachbarte Verschlusselemente 16 ein und untergreifen deren Köpfe 16' teilweise. Zum Einführen der Schweißvorsprünge 36, 38 zwischen die Verschlusselemente 16 können die Verschlusselemente 16 ggf. elastisch (nicht zerstörend) zur Seite hin verdrängt werden.

Es sei angemerkt, dass unterschiedlich weite Schweißspalte s1 und s2 alternativ durch eine Strukturierung der Mantelfläche der Sonotrode erhalten werden könnten (nicht näher dargestellt). Beispielsweise könnten in Umfangsrichtung umlaufende Vertiefungen und/oder Erhöhungen an der Mantelfläche der Sonotrode ausgebildet sein. Die ersten und zweiten Schweißvorsprünge können dabei denselben oder einen unterschiedlichen wirksamen Radius aufweisen.

Strukturierungen der Mantelfläche der Sonotrode 34 oder unterschiedlich gestaltete erste und zweite Schweißvorsprünge 36, 38 am Amboss 32 können auch den Energieeintrag in die ersten und zweiten Schweißstellen 18, 20 beeinflussen.

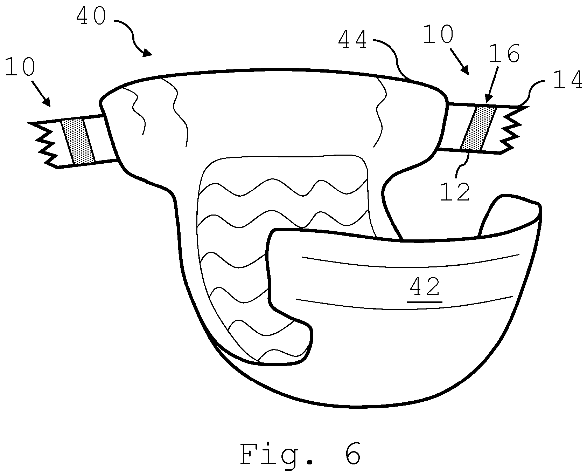

**Fig. 6** zeigt einen Hygieneartikel **40** in Form einer Windel mit zwei Verschlusssystemen 10, insbesondere Verschlusssystemen 10 in einer der zuvor beschriebenen Ausführungsformen. Das Verschlusssystem 10 weist jeweils ein Festlegeteil 14 und ein Trägerteil 12 mit vorstehenden Verschlusselementen 16 auf. Die Verschlusselemente 16 eignen sich zum Verhaken mit einem Vliesstoff des Hygieneartikels 40, insbesondere mit einem Vliesstoff einer "Landing zone" **42** des Hygieneartikels 40. Das Festlegeteil 14 ist jeweils an einem Windelohr **44** angebracht. Insbesondere kann das Festlegeteil 14 mittels einer Verschweißung und/oder Verklebung an dem Windelohr 44 befestigt sein. Vorteilhafterweise ist ein mittels des Verschlusssystems 10 erzeugter Verschluss der Windel mehrfach manuell lösbar und wieder einrichtbar. Auch das jeweilige Festlegeteil 14 kann aus Vliesstoff bestehen, sodass die Verschlusselemente 16 eines Trägerteils 12 im offenen Zustand der Windel auf dem zugehörigen Festlegeteil 14 "geparkt" werden können, beispielsweise um ein ungewolltes Verhaken mit Kleidung einer ein Kleinkind wickelnden Person oder anderen Windeln in einer Packung zu vermeiden.

Unter Vornahme einer Zusammenschau der Figuren der Zeichnung betrifft die Erfindung ein Verschlusssystem 10, ein Verfahren zum Herstellen eines Verschlusssystems 10 sowie einen Hygieneartikel 40 mit einem Verschlusssystem 10. Das Verschlusssystem 10 weist ein Trägerteil 12 mit an dessen Vorderseite 12' vorstehenden Verschlusselementen 16 und ein Festlegeteil 14, das an der Rückseite des Trägerteils 12 angeordnet ist, auf. Das Trägerteil 12 und das Festlegeteil 14 sind durch zumindest eine erste Schweißstelle 18 und zumindest eine zweite Schweißstelle 20 miteinander verbunden, wobei die Schweißstellen 18, 20 jeweils unterschiedlich ausgebildet sind. Die jeweilige unterschiedliche Ausbildung der Schweißstellen 18, 20 wird über ein Verschweißen, insbesondere mittels eines Ultraschallschweißverfahrens, mit jeweils unterschiedlichen Schweißparametern erreicht. Durch die unterschiedlichen Schweißparameter, insbesondere unterschiedlich weite Schweißspalte zwischen einem Amboss und einer Sonotrode, und die Anordnung der verschiedenen Schweißstellen (am Rand als auch in der Fläche) kann das mechanische Verhalten des Verschlusssystems wirksam beeinflusst werden. Insbesondere können mehrere erste und/oder zweite Schweißstellen 18, 20 in Zwischenräumen 24 zwischen Reihen 17 von Verschlusselementen 16 angeordnet sein. Zweite Schweißstellen 20 können insbesondere als "Flexzonen" ausgebildet sein, wobei sie eine geringere Biegesteifigkeit als erste Schweißstellen 18 aufweisen können. Das erfindungsgemäße Verfahren eignet sich insbesondere zum Herstellen eines erfindungsgemäßen Verschlusssystems 10. Der erfindungsgemäße Hygieneartikel 40 ist insbesondere in Form einer Windel ausgestaltet und weist wenigstens ein erfindungsgemäßes Verschlusssystem 10 auf.

**Bezugszeichenliste**

| | | | |
|---|---|---|---|
| 10 | Verschlusssystem | 28 | Längsmittelachse |
| 12 | Trägerteil | 30 | Ultraschallschweißanlage |
| 12' | Vorderseite | 32 | Amboss |
| 14 | Festlegeteil | 34 | Sonotrode |
| 15 | Querreihe | 36 | erster Schweißvorsprung |
| 16 | Verschlusselement | 38 | zweiter Schweißvorsprung |
| 16' | Kopf | 40 | Hygieneartikel |
| 16" | Stiel | 42 | Landing zone |
| 17 | Reihe | 44 | Windelohr |
| 17' | äußerste Längsreihe | | |
| 18 | erste Schweißstelle | d1 | erste Dicke |
| 20 | zweite Schweißstelle | d2 | zweite Dicke |
| 22 | Rand | s1 | erster Schweißspalt |
| 22' | Außenkante | s2 | zweiter Schweißspalt |
| 24 | Zwischenraum | MD | Maschinenrichtung |
| 26 | Querrand | | |

## Patentansprüche

1. Verschlusssystem (10) aufweisend ein Trägerteil (12) mit vorstehenden Verschlusselementen (16), die auf dessen Vorderseite (12') angeordnet sind, und ein Festlegeteil (14), das an der Rückseite des Trägerteils (12) angeordnet ist, wobei das Trägerteil (12) und das Festlegeteil (14) durch wenigstens eine erste Schweißstelle (18) und wenigstens eine zweite Schweißstelle (20) bereichsweise miteinander verbunden sind,
wobei die erste und zweite Schweißstelle (18, 20) durch Schweißen mit unterschiedlichen Schweißparametern erhalten sind.

2. Verschlusssystem (10) nach Anspruch 1, wobei sich wenigstens eine Materialeigenschaft der ersten und zweiten Schweißstelle (18, 20) voneinander unterscheidet.

3. Verschlusssystem (10) nach Anspruch 2, wobei sich eine Festigkeit der ersten und zweiten Schweißstelle (18, 20) voneinander unterscheidet.

4. Verschlusssystem (10) nach Anspruch 2 oder 3, wobei sich eine Steifigkeit, insbesondere eine Biegesteifigkeit der ersten und zweiten Schweißstelle (18, 20) voneinander unterscheidet.

5. Verschlusssystem (10) nach einem der vorhergehenden Ansprüche, wobei im Bereich der zweiten Schweißstelle (20) das Trägerteil (12) und das Festlegeteil (14) ein einheitliches Verbundmaterial bilden.

6. Verschlusssystem (10) nach einem der vorhergehenden Ansprüche, wobei im Bereich der ersten Schweißstelle (18) das Trägerteil (12) und das Festlegeteil (14) an ihren freien Oberflächen voneinander unterscheidbar bleiben.

7. Verschlusssystem (10) nach einem der vorhergehenden Ansprüche, wobei sich eine Tiefe des beim Verschweißen der ersten und zweiten Schweißstelle (18, 20) aufgeschmolzenen Bereichs voneinander unterscheidet.

8. Verschlusssystem (10) nach einem der vorhergehenden Ansprüche, wobei sich eine Dicke (d1, d2) des Verbundes aus Trägerteil (12) und Festlegeteil (14) im Bereich der ersten und zweiten Schweißstelle (18, 20) voneinander unterscheidet.

9. Verschlusssystem (10) nach einem der vorhergehenden Ansprüche, wobei die erste und/oder die zweite Schweißstelle (18, 20) durch Ultraschallschweißen erhalten ist/sind.

10. Verschlusssystem (10) nach einem der vorhergehenden Ansprüche, wobei sich die erste und/oder die zweite Schweißstelle (18, 20) zwischen benachbarten Reihen (17) von Verschlusselementen (16) erstrecken, insbesondere ohne die Verschlusselemente (16) zu beschädigen.

11. Verschlusssystem (10) nach einem der vorhergehenden Ansprüche, wobei mehrere voneinander beabstandete erste Schweißstellen (18) vorhanden sind, und insbesondere wobei mehrere voneinander beabstandete zweite Schweißstellen (20) vorhanden sind.

12. Verschlusssystem (10) nach einem der vorhergehenden Ansprüche, wobei die zweite(n) Schweißstelle(n) (20) in einer Längsrichtung am Trägerteil (12) durchgehend ausgebildet ist/sind.

13. Verschlusssystem (10) nach einem der vorhergehenden Ansprüche, wobei die ersten und zweiten Schweißstellen (18, 20) in einer Längsrichtung abwechselnd aufeinanderfolgen, insbesondere wobei die ersten und zweiten Schweißstellen (18, 20) unmittelbar aneinander angrenzen.

14. Verfahren zum Herstellen eines Verschlusssystems (10), insbesondere eines Verschlusssystems (10) nach einem der vorhergehenden Ansprüche, wobei einem Trägerteil (12) mit auf seiner Vorderseite (12') vorstehenden Verschlusselementen (16) auf seiner Rückseite ein Festlegteil (14) zugeführt wird, wobei das Trägerteil (12) und das Festlegteil (14) durch zumindest eine erste und zumindest eine zweite Schweißstelle (18, 20) bereichsweise miteinander verbunden werden, und wobei die erste und zweite Schweißstelle (18, 20) mit unterschiedlichen Schweißparametern eingebracht werden.

15. Verfahren nach Anspruch 14, wobei die erste und/oder zweite Schweißstelle (18, 20) derart eingebracht wird/werden, dass sie sich über zumindest ein Verschlusselement (16) hinweg erstreckt/erstrecken, insbesondere sodass das zumindest eine Verschlusselement (16) beim Schweißen zerstört wird.

16. Verfahren nach Anspruch 14 oder 15, wobei die erste und/oder zweite Schweißstelle (18, 20) derart eingebracht wird/werden, dass sie sich zwischen benachbarten Reihen (17) von Verschlusselementen (16) erstreckt/erstrecken, insbesondere ohne die Verschlusselemente (16) zu beschädigen.

17. Verfahren nach einem der Ansprüche 14 bis 16, wobei die erste und zweite Schweißstelle (18, 20) in einem Arbeitsgang eingebracht werden.

18. Verfahren nach einem der Ansprüche 14 bis 17, wobei die erste und zweite Schweißstelle (18, 20) durch Ultraschallschweißen eingebracht werden, insbesondere wobei eine Sonotrode (34) an dem Festlegeteil (14) anliegt und ein Amboss (32), insbesondere ein rotierender, walzenförmiger Amboss (32), an dem Trägerteil (12) anliegt.

19. Verfahren nach Anspruch 18, wobei der Amboss (32) wenigstens einen ersten und wenigstens einen zweiten Schweißvorsprung (36, 38) zum Erzeugen der ersten und zweiten Schweißstelle (18, 20) aufweist.

20. Verfahren nach Anspruch 19, wobei sich eine Höhe des ersten und zweiten Schweißvorsprungs (36, 38) voneinander unterscheidet bzw. wobei sich ein Radius des ggf. walzenförmigen Ambosses (32) im Bereich des ersten und zweiten Schweißvorsprungs (36, 38) unterscheidet.

21. Verfahren nach einem der Ansprüche 18 bis 20, wobei ein Schweißspalt (s1, s2) zwischen dem Amboss (32) und der Sonotrode (34) im Bereich der ersten und zweiten Schweißstelle (18, 20) unterschiedlich weit ist.

22. Hygieneartikel (40), insbesondere Windel, mit einem Verschlusssystem (10) nach einem der Ansprüche 1 bis 13.
